# EUROPEAN PATENT APPLICATION

(11) **EP 4 729 626 A1**
(43) Date of publication of application: **22.04.2026**
(21) Application number: 24823724.0
(22) Date of filing: 13.06.2024
(51) Int. Cl.: C12Q 1/22

(54) **RAPID STERILITY TEST METHOD AND RAPID STERILITY TEST PLATFORM FOR VERIFYING SAFETY OF BIOPHARMACEUTICALS**

(30) Priority: 14.06.2023 KR 20230076196
(71) Applicant: Seoul National University R&DB Foundation, Seoul 08826 (KR); Seoul National University Hospital, Seoul 03080 (KR)
(72) Inventor: KWON, Sunghoon, Seoul 06101 (KR); KANG, Jun Won, Seoul 08275 (KR); KIM, Hamin, Seoul 08832 (KR); JANG, Hae Wook, Seoul 08788 (KR); KIM, Tae Hyun, Jinju Gyeongsangnam-do 52849 (KR); JOO, Hyelyn, Namyangju-si Gyeonggi-do 12251 (KR); LEE, Giyoon, Seoul 08832 (KR); LEE, Eunju, Seoul 03080 (KR)
(74) Representative: Prüfer & Partner mbB Patentanwälte · Rechtsanwälte
(86) International application number: PCT/KR2024/008125
(87) International publication number: WO 2024/258207

(57) **Abstract**

This invention relates to an integrated system and methodology for drastically reducing the time required for sterility testing used to verify safety during the manufacturing process of biopharmaceuticals. More specifically, the invention provides a rapid sterility test method and a rapid sterility test platform for demonstrating the safety of biopharmaceuticals, comprising magnetic nanoparticles coated with a protein capable of binding to microorganisms, a biochip specialized for nanoparticle-based analysis, an automated imaging device and analysis software, and the procedures and methods for performing rapid sterility testing.

## Description

### [Technical Field]

The present invention relates to a rapid sterility testing method and a rapid sterility testing platform for demonstrating the safety of biopharmaceuticals, and more particularly, to an integrated system and methodology capable of remarkably shortening the time required for a sterility test for safety verification during the manufacturing process of biopharmaceuticals. Specifically, the present invention relates to a rapid sterility testing method and a rapid sterility testing platform for demonstrating the safety of biopharmaceuticals, which include magnetic particles coated with proteins capable of binding to microorganisms, a biochip specialized for microorganism analysis in combination with nanoparticles, automated imaging equipment and analysis software, and procedures and methods by which rapid sterility testing is performed.

### [Background Art]

The sterility test is one of the test methods for pharmaceutical quality control, which is a test for detecting bacteria and fungi, and is applied to raw materials and preparations during the manufacture of sterile pharmaceuticals. In this test, a prescribed amount of a sample is taken, and the presence or absence of bacteria and fungi presumed to originate from the sample is examined by carrying out the experiment using a specified medium in a specified manner. Currently, there are two types of sterility tests for pharmaceuticals: the membrane filtration method and the direct method, and the membrane filtration method is currently used as the standard. In the membrane filtration method, biopharmaceuticals are passed through a filter to purify and concentrate components other than microorganisms, after which the microorganisms are introduced into a microbial growth medium, and turbidity that increases as microorganisms divide is detected. This process typically requires a long period of about two weeks and is labor-intensive. However, in the case of most biopharmaceuticals, unlike conventional chemically synthesized pharmaceuticals, the shelf life is significantly shorter than two weeks. Therefore, in practice, administration to patients is prioritized immediately after manufacturing, and the results of the sterility test are only obtained afterward, which poses a clinical risk of causing infections due to microorganisms. Furthermore, biopharmaceuticals, including cell therapy products, are manufactured in a patient-specific manner, making standardized management difficult. As advanced pharmaceuticals, their regulatory frameworks have only recently been established, and rapid bacterial/fungal testing methods suitable for sterility testing of cell therapy products have not yet been developed, thus microbial safety management remains a limiting factor for both manufacturers and patients. Consequently, as the use of biopharmaceuticals continues to increase, there is a growing need for the development of a rapid sterility testing method that addresses the limitations of existing sterility testing methods which require long detection times.

To address this issue, methods that detect microorganisms by omitting the cultivation step have been proposed in order to reduce the time required for sterility testing. Representative technologies include solid phase cytometry and polymerase chain reaction (PCR). In solid phase cytometry, biopharmaceuticals are passed through a membrane filter, microorganisms trapped on the filter are stained, and detection is performed using a microscope. In PCR, detection is carried out by using primers specific to microorganisms of interest, and if the microorganism is present, a signal is amplified. Although both methods offer the advantage of detecting microorganisms within approximately three hours, they are difficult to apply universally across various types of microorganisms and biopharmaceuticals, and the occurrence rates of false positives and false negatives remain high, making them unsuitable as replacements for conventional sterility testing of biopharmaceuticals. Accordingly, current technological developments are focused on methods that detect metabolites produced during microbial growth, such as carbon dioxide or adenosine triphosphate (ATP). However, these methods also suffer from the limitation that biopharmaceutical components and microorganisms cannot be effectively separated due to the purification efficiency limitations of the membrane filtration method. As a result, interference and false positives occur due to metabolites produced from non-microbial cells, and approximately five days are required to obtain meaningful signal measurements. The present invention addresses these problems by providing a methodology and integrated system capable of separating microorganisms from biopharmaceuticals, in which cells and microorganisms are mixed, with a purification and concentration efficiency superior to the membrane filtration method, and rapidly detecting microbial metabolites with high specificity.

### [Disclosure]

### [Technical Problem]

In order to solve the problems described above, the present invention aims to provide an integrated system and methodology capable of remarkably shortening the time required for a sterility test for safety verification during the manufacturing process of biopharmaceuticals. Specifically, the present invention relates to a rapid sterility testing method and a rapid sterility testing platform for demonstrating the safety of biopharmaceuticals, which include magnetic particles coated with proteins capable of binding to microorganisms, a biochip specialized for microorganism analysis in combination with nanoparticles, automated imaging equipment and analysis software, and procedures and methods by which rapid sterility testing is performed.

### [Technical Solution ]

In order to solve the above-described problems, the present invention provides a rapid sterility testing method using a biochip including one or more chambers into which a sample and a control are respectively loaded, and one or more magnets installed at one side of each chamber, the method comprising: mixing magnetic particles with a test subject material to bind a target analyte to the magnetic particles; separating the magnetic particles from the test subject material to prepare a sample; and determining the presence of the target analyte by injecting the separated sample and an indicator substance into the chamber of the biochip and microbial culture, wherein the step of determining the presence of the target analyte comprises determining whether the target analyte is present in the sample by comparing fluorescence or color of images of the sample and the control after microbial culture.

In one embodiment, the test subject material comprises a biopharmaceutical, a cell therapy product, a clinical specimen, a chemical pharmaceutical product, a cosmetic, a food, or an environmental sample.

In one embodiment, the target analyte comprises a bacteria, mycoplasma, or fungi.

In one embodiment, the preparing the sample comprises concentrating the target analyte bound to the magnetic particles on a growth medium.

In one embodiment, the growth medium comprises Tryptic Soy Broth, Muller-Hinton Broth, Fluid Thioglycollate Medium, Sabouraud Broth, or Luria-Bertani Broth.

In one embodiment, the determining the presence of the target analyte comprise: spacing the chamber from the magnet; injecting an indicator substance, sample, and control into the chamber and microbial culture for a predetermined time; approaching the chamber to the magnet after completion of microbial culture; and acquiring an image of the inside of the chamber and comparing acquired images of the separated sample and the control.

In one embodiment, the target analyte is bound to the magnetic particles to form a complex, and the complex is moved to one side of the chamber by the magnet when the chamber is brought into proximity with the magnet.

In one embodiment, the microbial culture is performed for 4 to 48 hours.

In one embodiment, image acquisition is performed at intervals of 10 to 120 minutes.

In one embodiment, the indicator substance undergoes fluorescence or color change by the target analyte.

In one embodiment, the indicator substance is a redox indicator substance, a pH indicator, a dye-based reagent, an enzyme reaction indicator, or an antibody-based reagent.

The present invention also provides a biochip for a rapid sterility testing platform, comprising: a lower plate including chambers into which the control and the sample are loaded; and an upper plate engaged with the lower plate and including a magnet, wherein the lower plate comprises: a bottom surface; a central axis protruding upward from the bottom surface; and one or more chambers installed at a predetermined distance from the central axis, and the upper plate comprises: a central axis insertion hole into which the central axis of the lower plate is inserted; a chamber insertion hole into which the chamber is inserted; and a magnet installed at one side of the chamber insertion hole.

In one embodiment, the chamber comprises one or more sample chambers and one or more control chambers.

In one embodiment, the chambers are installed at equal intervals at a uniform distance from the central axis.

In one embodiment, the chamber further contains magnetic particles and an indicator substance.

In one embodiment, the magnetic particles form a complex by binding with the target analyte.

In one embodiment, the magnetic particles include a functional group or substance capable of binding to microorganisms on their surface.

In one embodiment, the complex is positioned at one side of the chamber by the magnet during fluorescence or optical observation using the indicator substance.

In one embodiment, the upper plate is rotated at a predetermined angle around the central axis.

In one embodiment, the control chamber insertion hole has an arc shape around the central axis.

In one embodiment, the distance between the magnet and the chamber changes according to rotation of the upper plate.

In one embodiment, a rotation means for rotating the upper plate is installed on an outer side surface of the upper plate.

The present invention also provides a rapid sterility testing platform comprising the biochip for the rapid sterility testing platform.

In one embodiment, the rapid sterility testing platform comprises: the biochip; a rotational drive unit for rotating the upper plate of the biochip; an illumination unit installed above or below the biochip; and an image acquisition unit for capturing the inside of the chamber of the biochip.

In one embodiment, the image acquisition unit acquires fluorescence images or optical images of the inside of the chamber.

In one embodiment, the rapid sterility testing platform determines the presence of microorganisms in the sample by comparing fluorescence or color between an initially acquired image and a current image.

### [Advantageous Effects ]

The rapid sterility testing method proposed in the present invention reduces the sterility test time, which previously required 14 days, to within one day (24 hours), thereby enabling the rapid assurance of the safety of biopharmaceuticals. Considering that biopharmaceuticals manufactured using human-derived materials such as cells and proteins have short shelf lives and must be administered to patients promptly, the reduction in total testing time is advantageous for preserving the quality of the biopharmaceuticals at the time of administration, while also improving therapeutic outcomes for patients through faster administration. In addition, from social and industrial perspectives, the reduction of the safety verification period by 13 days or more enables significant savings in logistics costs.

In the conventional sterility testing method, contamination must be continuously monitored at specific intervals, such as 24, 48, and 72 hours after microbial culture, for up to 14 days. In contrast, the rapid sterility testing method of the present invention enables real-time or approximately 30-minute interval continuous imaging within the system to determine contamination status. Therefore, the temporal resolution is significantly higher, and more accurate analysis is possible based on fluorescence intensity measurements. Additionally, since automated systems eliminate the need for manual observation at fixed intervals, the labor-intensive workload and human resource requirements are greatly reduced compared to conventional methods.

From a technical perspective, both conventional sterility testing methods and recently developed technologies use the membrane filtration method to concentrate contaminated microorganisms from biopharmaceutical samples. For sterility confirmation, detection must be universally applicable to microorganisms ranging from bacteria with sizes around 1 µm to fungi with sizes around 10 µm. However, in the filtration method, losses of microorganism samples may occur depending on pore size. When a filter with a small pore size is used, components such as cells and proteins present in the biopharmaceutical are also concentrated, thereby reducing the sensitivity of observing microorganisms or microbial metabolites during subsequent analysis. In the present invention, microorganisms are specifically separated and concentrated regardless of size through functionalized nanoparticles capable of specifically binding to microorganisms present in biopharmaceuticals. As a result, the present invention enables highly sensitive detection after microbial concentration, compared to the conventional filtration method. Therefore, the concentration of microorganisms in a small volume promotes the reaction of resazurin staining reagent, enabling faster metabolic measurement than prior techniques. Notably, viability testing that detects metabolites (representatively ATP, CO₂, NADH), which is recommended as the standard test for sterility confirmation to target all species of microorganisms, is hindered by metabolites produced by non-microbial cells present in biopharmaceuticals. Therefore, the separation and concentration method utilized in the present invention provides the advantage of significantly improving analytical specificity.

The nanoparticle-based microorganism separation and concentration method may also be applied not only to Resazurin-based viability reagent analysis but also to molecular diagnostic methods such as PCR, next-generation sequencing, and advanced technologies such as flow analyzers. The characteristics of the invention allow flexible adaptation of the technique depending on its intended use. For example, if contamination is confirmed in a biopharmaceutical sample, molecular diagnostics may be used to identify the species of the microorganism in order to trace contamination pathways or prevent secondary contamination. Alternatively, the method may be applied to samples requiring sterility verification in food, cosmetics, or environmental monitoring depending on the purpose.

The invention of the chip specialized for nanoparticle-based analysis allows stable observation of microorganism signals by preventing inhibition of microbial growth among microorganisms separated and concentrated by binding to nanoparticles. If microorganisms bound to nanoparticles remain aggregated on the wall of the biochip, normal growth cannot occur. However, the biochip included in the present invention enables the application or removal of magnetic force through a simple action, allowing normal microbial growth. Therefore, compared to systems without the chip, the production of microbial metabolites increases, enabling faster and more sensitive detection of microorganisms or microbial metabolites.

Continuous fluorescence imaging over time is performed using a miniaturized imaging system of 224 mm × 120 mm × 140 mm, as proposed in the present invention. The imaging system includes an imaging area for the chip sample, an xyz motorized stage for adjusting the imaging position and focus, a light source for fluorescence imaging, a 4× objective lens, and a CCD camera, and is manufactured in a simplified form optimized for the purpose of rapid sterility testing method. This configuration enables industrial deployment in a much more cost-effective and simplified form compared to commercially available equipment such as platereaders, and its compact design allows effortless placement inside an incubator, providing advantages in temperature control and spatial optimization.

### [Description of Drawings]

FIG. 1 illustrates the configuration and operating principle of the biochip for a rapid sterility testing platform according to an embodiment of the present invention.
FIG. 2 illustrates the shape of the biochip for a rapid sterility testing platform according to an embodiment of the present invention, wherein (a) shows the overall shape, (b) shows the position of the chamber during reverse rotation, and (c) shows the position of the chamber during forward rotation.
FIG. 3 illustrates the configuration of an upper plate of the biochip for a rapid sterility testing platform according to an embodiment of the present invention.
FIG. 4 illustrates a rapid sterility testing platform according to an embodiment of the present invention.

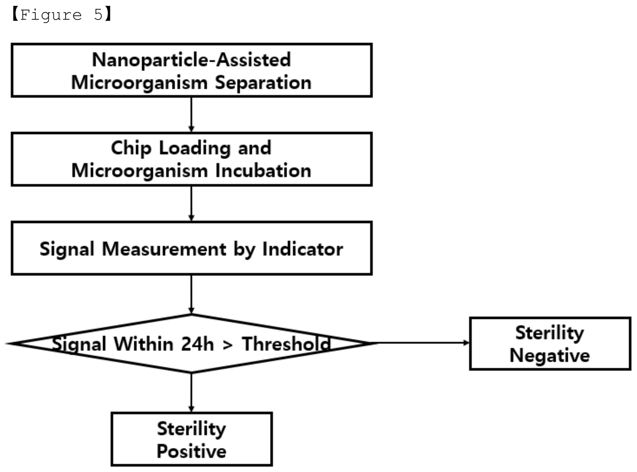

FIG. 5 illustrates a rapid sterility testing method using the rapid sterility testing platform according to an embodiment of the present invention.
FIG. 6 illustrates a concentration method using magnetic nanoparticles according to an embodiment of the present invention.
FIG. 7 illustrates the results of microbial growth depending on magnetic properties according to an embodiment of the present invention.
FIG. 8 compares the rapid sterility testing method according to an embodiment of the present invention with a conventional sterility testing method.
FIG. 9 illustrates rapid sterility testing results for various biopharmaceuticals according to an embodiment of the present invention.
FIG. 10 illustrates microorganism contamination determined by visual observation according to an embodiment of the present invention.

### [Best Mode]

Hereinafter, preferred embodiments of the present invention will be described in detail. In describing the present invention, if it is determined that a detailed description of related known technologies may obscure the gist of the present invention, the detailed description thereof will be omitted. Throughout the specification, it is to be understood that the singular forms comprise plural referents unless the context clearly dictates otherwise, and it is to be understood that the terms such as "comprise" or "have" as used in the present specification are intended to designate the presence of stated features, numbers, steps, operations, components, parts or combinations thereof, but not to preclude the possibility of the presence or addition of one or more other features, numbers, steps, operations, components, parts, or combinations thereof. In addition, in performing the method or preparation method, each process constituting the method may occur in a different order from the specified order unless a specific order is clearly described in context. That is, each process may occur in the same order as specified, may be performed substantially simultaneously, or may be performed in the reverse order.

The technology disclosed in this specification is not limited to the embodiments described herein and may be embodied in other forms. However, the embodiments introduced herein are provided so that the content disclosed herein may be thorough and complete, and the technical spirit of the present technology may be sufficiently understood by those skilled in the art. In the drawings, in order to clearly express the components of each device, the size of the components, such as width or thickness, is shown somewhat enlarged. Overall, when describing the drawings, it was described from the observer's point of view, and when one element is referred to as being located on another element, this comprises all meanings that one element may be located directly on another element or additional elements may be interposed between them. In addition, those skilled in the art will be able to implement the spirit of the present invention in various other forms within the scope that does not depart from the technical spirit of the present invention. In addition, the same reference numerals on a plurality of drawings refer to elements that are substantially the same as each other.

In this specification, the term 'and/or' comprises a combination of a plurality of recited items or any one of a plurality of recited items. In this specification, 'A or B' may comprise 'A', 'B', or 'both A and B'.

The present invention relates to a rapid sterility testing method using a biochip comprising one or more chambers in which a sample and a control are respectively loaded, and one or more magnets installed at one side of each chamber; the method comprising: mixing magnetic particles with a test subject material to bind a target analyte to the magnetic particles; separating the magnetic particles from the test subject material to prepare a sample; and determining the presence of the target analyte by injecting the separated sample and an indicator substance into the chamber of the biochip and microbial culture, wherein the step of determining the presence of the target analyte comprises determining whether the target analyte is present in the sample by comparing fluorescence or color of images of the sample and the control after microbial culture.

In the case of biological preparations such as biopharmaceuticals, the presence of undesirable microorganisms can significantly reduce safety. The present invention provides a rapid sterility testing method capable of determining the presence of microorganisms at a faster rate than conventional methods, thereby verifying the safety of such pharmaceuticals.

The biochip of the present invention comprises one or more chambers in which the sample and the control are respectively loaded, and one or more magnets installed at one side of each chamber. The structure of such a biochip will be described later.

Magnetic particles may be mixed with the test substance to bind the target analyte to the magnetic particles. The magnetic particles refer to particles that bind to a target analyte present in the test substance and comprise paramagnetic or ferromagnetic material inside. This allows the target analyte to be separated from the test substance and enables easy movement of the magnetic particles by using magnetism. The target analyte may form a complex by binding to the magnetic particles. As described below, the surface of the magnetic particles comprises a functional group or substance capable of binding to microorganisms, allowing selective binding with the target analyte to form a complex.

The magnetic particles may be those conventionally used for microbial separation without limitation, and may be manufactured in micro-particle or micro-rod form. The magnetic particles may also be magnetic nanoparticles having a nanoscale size.

The magnetic particles comprise, on the surface, a functional group or substance capable of binding to microorganisms. Through this, selective binding with an target analyte present in the test substance can be achieved. The functional group or substance may be beta-2-glycoprotein 1, mannose binding lectin, an antibody, an aptamer, an antibiotic, or a polymer.

The test substance refers to a material requiring microbial safety, and may specifically be a biopharmaceutical, cell therapy product, clinical specimen, chemical drug, cosmetic, food, or environmental sample. In particular, the biopharmaceutical may be produced based on proteins, peptides, antibodies, or vaccines, or based on genes, stem cells, human-derived cells, human cells or tissues, and the same applies to clinical specimens and cell therapy products. These materials can cause fatal harm to the human body if contaminated with microorganisms, and therefore ensuring safety is essential.

The target analyte refers to microorganisms that may reduce the safety of the test substance, particularly pharmaceuticals, and may be bacteria, mycoplasma, or fungi. If such microorganisms are found in a pharmaceutical, the pharmaceutical is considered contaminated, and due to the nature of pharmaceuticals, contamination generally results in disposal of not only the affected product but also entire lots or batches.

After the target analyte is bound to the magnetic particles as described above, the magnetic particles may be separated from the test substance. Since the target analyte is bound to the magnetic particles, such separation may simply be performed through washing. After washing, a magnet may be used to remove the washing solution and impurities. Since the target analyte is separated using magnetic particles, the magnetic particles and the target analyte bound thereto can be easily moved by a magnet. For example, a magnetic rod may be inserted into the washing container to collect the magnetic particles, or a magnet may be brought near the side or underside of the washing container to hold the magnetic particles in place while the liquid is removed. This enables separation without loss of magnetic particles and allows recovery even when the target analyte concentration is very low, thereby greatly reducing the probability of false negatives.

Furthermore, it is preferable to concentrate the target analyte bound to the magnetic particles on a growth medium. Concentration on the growth medium may be achieved by fixing the magnetic particles to the side of the container by a magnet, removing the supernatant, and re-suspending in growth medium. Depending on container size and amounts of magnetic particles, an appropriate amount of growth medium may be used, preferably about 100 µL. To effectively recover magnetic particles on the container wall, it is preferable to wash at least three times with the growth medium. The growth medium promotes growth of the target analyte, reducing detection time, and concentration into a small volume further reduces detection time.

The growth medium may be any medium capable of microbial culture the target analyte, but is preferably selected from Tryptic Soy Broth, Mueller-Hinton Broth, Fluid Thioglycollate Medium, Sabouraud Broth, or Luria-Bertani Broth.

The separated magnetic particles and the indicator may then be introduced into the chambers of the biochip and cultured to determine the presence of the target analyte. The biochip used at this time comprises one or more chambers in which the sample and the control are respectively loaded, and one or more magnets installed on one side of each chamber, as described later.

The step of determining the presence of the analyte may include: spacing the chamber from the magnet; injecting an indicator substance, sample, and control into the chamber and microbial culture for a predetermined time; approaching the chamber to the magnet after completion of microbial culture; and acquiring an image of the inside of the chamber and comparing acquired images of the separated sample and the control.

The chamber may be spaced from the magnet. As described above, when the analyte bound to the magnetic particles is cultured, it is preferable that the complex formed by the binding of the magnetic particle floats in the chamber. Therefore, by spacing the chamber from the magnet, the magnetic attractive force can be reduced, and the complex can freely float inside the chamber.

The indicator substance, the sample, and the control may be injected into the chamber and incubated for a predetermined time. The sample may be, as described above, a material separated from a test target for sterility testing, and may be a complex in which the analyte is bound to the surface of the magnetic particles.

The control refers to a substance whose safety has been verified, i.e., a negative control, and preferably refers to a specimen having the same composition as the sample except that the analyte is excluded. That is, the control may include magnetic particles that do not form a complex and the same growth medium as the sample. By simultaneously incubating the control and the sample and observing changes in the chamber, the presence of the analyte in the chamber can be determined.

In addition, the sample may be prepared separately from the biochip as described above, but may also be concentrated and used inside the biochip. In this case, the biochip includes the magnetic particles therein, and the test target material may be supplied into the biochip, after which a washing process may be performed using the magnet provided in the biochip. In this washing process, the complex may be collected to one side of the chamber by the magnet, and the washing solution and foreign substances may be removed as described above.

That is, when the sample is prepared outside the biochip, a separate chamber or container may be used, and when the sample is prepared using the biochip, the internal chamber of the biochip may be used to prepare the sample.

The indicator substance is used to confirm the presence of the analyte forming a complex with the sample during incubation in the chamber, and may be a redox indicator, a pH indicator, a dye-based reagent, an enzyme reaction indicator, or an antibody-based reagent. These indicators detect chemical changes in the chamber associated with microbial respiration, digestion, or reproduction, causing changes in color or fluorescence, thereby allowing the presence or absence of microorganisms in the sample chamber to be confirmed.

The indicator substance may be injected before or after injection of the sample and control, but may also be supplied in a state preloaded inside the biochip. In such a case, user convenience is greatly improved; however, the biochip containing the indicator substance must be exchanged depending on the sample type, which may increase analysis cost. Therefore, the injection timing of the indicator substance may be appropriately adjusted depending on user circumstances and the surrounding environment.

The redox indicator refers to a substance in which color or fluorescence changes through oxidation or reduction reactions, and is preferably resazurin. Generally, all cells containing microorganisms perform various oxidation and reduction reactions internally. When the redox indicator is present, the color or fluorescence may change due to such reactions. In particular, resazurin has cell permeability, and thus such changes in color or fluorescence are more noticeable when redox reactions occur inside cells.

Resazurin is a phenoxazine dye having non-toxicity, cell permeability, and redox sensitivity, and changes from blue to red upon reaction with living cells, while also exhibiting fluorescence, making it measurable by optical or fluorescence observation. Thus, when such a color change occurs, it can be confirmed that living cells are present in the sample, and if no fluorescence or color change occurs even after incubation, sterility can be confirmed.

The sample and control may be injected into the chamber and incubated for a predetermined period of time. Preferably, the incubation is carried out for 4 to 48 hours. In conventional sterility tests, turbidity caused by microorganisms is used for detection, which requires long-term incubation (about two weeks). However, in the present invention, the presence of microorganisms is detected by changes in the redox indicator, allowing determination within a short incubation period as described above. If incubation is less than 4 hours, changes may be difficult to observe, and if it exceeds 48 hours, the total test process becomes inefficient.

After the incubation is completed, the chamber may be moved closer to the magnet. In this process, the magnetic particles in the control chamber and the complexes (magnetic particles + microorganisms) in the sample chamber may be collected to one side of the chamber by the magnet.

When such collection occurs, the magnetic particles and microorganisms are positioned away from the center of the chamber, allowing clearer imaging of the chamber interior.

An image of the interior of the chamber may be captured, and the images obtained from the sample and the control may be compared. As described above, the control has verified sterility, and any changes may only be due to external environmental factors. Therefore, if the sample shows change identical to the control, it can be determined that no microorganisms are present in the sample. If the sample shows color or fluorescence changes, it can be determined that microorganisms are present.

The image acquisition is preferably performed at intervals of 10 to 120 minutes. Conventional tests required two weeks of incubation, and thus images were typically obtained on a daily (24-hour) basis. However, since the incubation period in the present invention is very short (4 to 48 hours), and the presence of microorganisms can be determined immediately upon color change, images may be acquired at intervals of 10 to 120 minutes, preferably 40 to 80 minutes, and most preferably 60 minutes.

The present invention also provides a biochip for a rapid sterility testing platform, comprising: a lower plate including chambers into which the control and the sample are loaded; and an upper plate engaged with the lower plate and including a magnet, wherein the lower plate comprises: a bottom surface; a central axis protruding upward from the bottom surface; and one or more chambers installed at a predetermined distance from the central axis, and the upper plate comprises: a central axis insertion hole into which the central axis of the lower plate is inserted; a chamber insertion hole into which the chamber is inserted; and a magnet installed at one side of the chamber insertion hole.

The lower plate is a structure on which the chambers are installed, and the control and the sample may be loaded into the chambers.

The bottom surface (110) of the lower plate is generally formed as a flat plate, but may be formed in various shapes depending on the arrangement of the chambers and the configuration of the rapid sterility testing platform. In the present invention, a circular plate shape is described as an example; however, the bottom surface may alternatively be formed in a square, oval, triangular, or polygonal shape, or may be formed with curved or contoured portions. Further, in addition to plate shapes, the bottom surface may also be formed as a columnar structure or a structure having a slope in one or more directions.

Preferably, the bottom surface (110) is formed as a square plate. When mounted in the rapid sterility testing platform, the square plate shape may be inserted into a corresponding groove without requiring an additional fixing member, preventing rotation of the lower plate when the upper plate rotates.

The lower plate includes the central axis (120) and the chambers.

The central axis (120) serves as the rotational center for the upper plate which is fitted onto the lower plate, and is installed to protrude upward from the bottom surface (110). The central axis (120) may preferably have a circular cross-section to allow smooth rotation of the upper plate.

Although the central axis (120) is preferably positioned at the center of the bottom surface (110), it may alternatively be positioned at an offset location depending on the required chamber layout or stabilization structure.

The central axis (120) may also include a separation-preventing means at the insertion end to prevent the upper plate from being detached after engagement. The separation-preventing means may be formed larger in diameter than the central axis insertion portion (210) of the upper plate. Additionally, the central axis (120) may be formed as a hollow pipe with a slit at its upper portion such that the diameter decreases when inserted through the central axis insertion portion (210), thereby improving ease of engagement.

Each chamber is a structure into which the sample and the control are loaded, having a closed lower end and an open upper end. The chamber is preferably formed as a hollow cylindrical tube structure, with the lower portion formed of a transparent material to facilitate image acquisition. The chamber may have a circular, oval, square, or polygonal cross-section, but is preferably circular to improve mixing efficiency and uniformity during microbial culture. That is, the chamber is most preferably formed as a cylindrical tube closed at the bottom and open at the top.

The upper opening of the chamber may simply remain open, but is preferably sealed with a polymer film. The polymer film prevents contamination after loading of the sample and the control, and may include a slit (e.g., cross-shaped "+" or "-"). The sample and control are typically loaded using a dropper, pipette, or syringe. Therefore, the sample and control can be loaded through the slit, and after loading is complete, the slit is closed elastically, thereby preventing contact with external contaminants, particularly microorganisms.

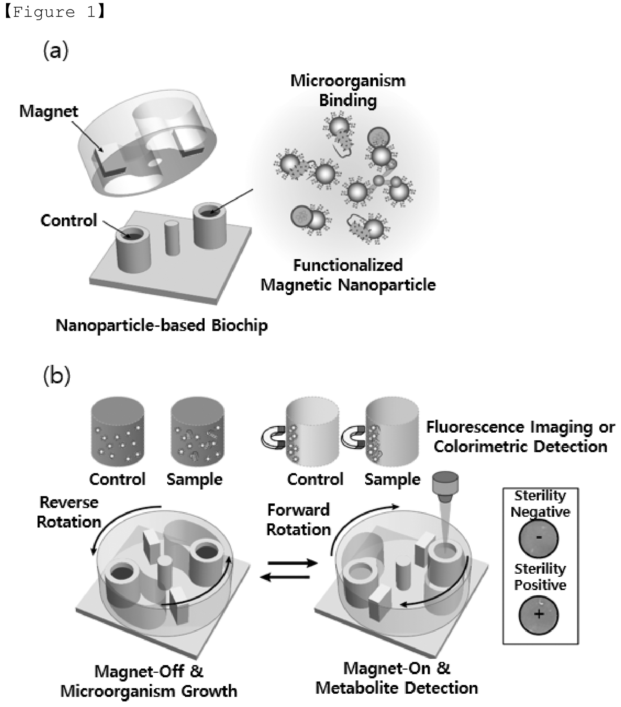

The chambers may include one or more sample chambers (131) and one or more control chambers (132). The sample chamber (131) is for the material requiring sterility testing, while the control chamber (132) contains a sterility-verified negative control solution (see Fig. 1(a)). By simultaneously microbial culture the sample and the control under identical conditions, contamination in the sample can be readily detected.

Although only one sample chamber (131) and one control chamber (132) may be installed, multiple chambers may also be installed. If multiple sample chambers (131) are installed, sterility testing can be performed simultaneously on multiple samples. If multiple control chambers (132) are installed, sterility testing can be performed under various conditions.

The chambers may be installed at equal intervals at a uniform distance from the central axis (120). As will be described below, the chambers are inserted into the chamber insertion portions, and the upper plate rotates thereafter. Therefore, in order to facilitate such rotation of the upper plate, it is preferable that the chambers are arranged to maintain a predetermined distance from the central axis (120). In addition, it is preferable that the chambers are installed at equal intervals so that the distance between each chamber and the magnet (230), which will be described later, is maintained uniformly. If the spacing between the chambers is not uniform, the distances between each chamber and the magnet (230) will differ, resulting in the magnetic particles inside each chamber being subjected to different magnetic forces, which may cause variations in the experimental results among the chambers.

The chamber may further contain magnetic particles and a redox indicator material.

The magnetic particles may be bound to microorganisms to form a complex (see FIG. 1(a)). During the forward rotation of the upper plate to be described below, as the magnet (230) approaches one side of the chamber, the magnetic particles may be moved toward a side wall of the chamber by magnetic force (see FIG. 1(b)). Through this, the microorganism-magnetic particle complexes can be removed from the central region of the chamber, thereby allowing a clearer image to be obtained. That is, the complex may be located at one side of the chamber by using the magnet (230) during fluorescent or optical observation using the indicator substance, which will be described below.

Microorganisms may be bound to the magnetic particles via known binding chemistry, but preferably the magnetic particle surfaces are functionalized with materials that selectively bind to proteins on the microbial surface. Such binding materials may include apolipoprotein H, beta-2-glycoprotein 1, mannose-binding lectin, antibodies, aptamers, antibiotics, or polymers.

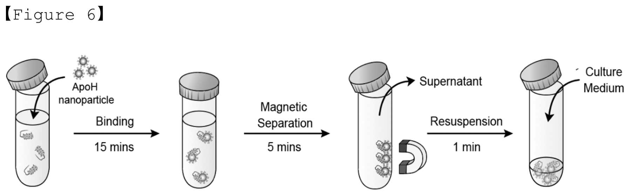

In addition, although the magnetic particles may be directly mixed with the sample and used as described above, a concentration process using the magnetic particles may also be performed (see FIG. 6). This concentration process may be carried out inside the chamber, or alternatively, it may be carried out in a separate chamber. That is, when the magnetic particles are mixed with a test sample and subsequently separated, the microorganisms (analytes) attached to the magnetic particles may also be separated. Using this approach, microorganisms can be isolated from the test sample at a high concentration, and the isolated microorganisms can then be cultured to verify sterility.

The indicator material is as previously described and its explanation is omitted.

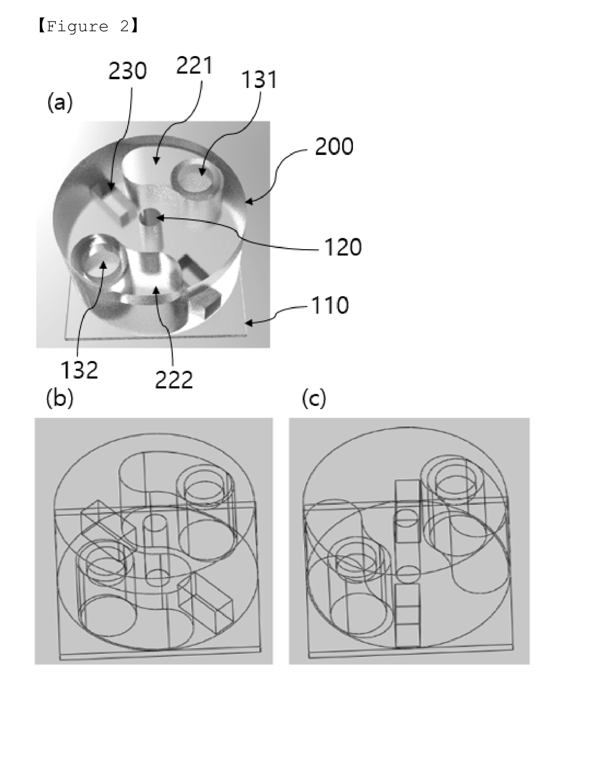

The upper plate is a portion that is fitted to the lower plate, and as described above, the central axis (120) formed on the lower plate may be inserted into the central axis insertion opening (210) provided on the upper plate to be coupled thereto. That is, the upper plate may be coupled such that it is rotatable about the central axis (120) (see FIGS. 2(b) and 2(c)). Since a magnet (230), which will be described below, is installed on the upper plate, the rotation of the upper plate may change the distance between the magnet (230) and the chamber of the lower plate. In this case, when the magnet (230) moves away, the magnetic nanoparticles inside the chamber may freely float in the chamber, whereas when the magnet (230) approaches, the magnetic nanoparticles may be collected to one side of the chamber by the magnet (230) (see FIG. 1(b)). Further details will be described below.

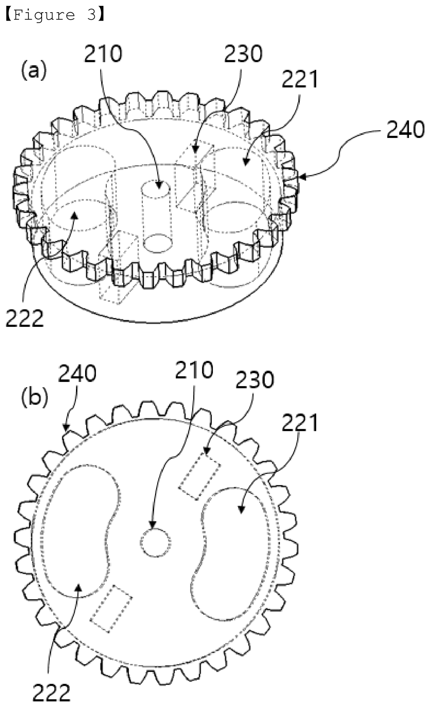

The upper plate includes the central axis insertion portion (210); the chamber insertion portion; and the magnet (230) positioned on one side of the chamber insertion portion (see Fig. 3).

The central axis insertion opening (210) is a portion into which the central axis (120) installed on the lower plate is inserted, and may be formed in a circular shape corresponding to the central axis (120) so that the central axis (120) can be easily rotated. In this case, it is preferable that the central axis insertion opening (210) be located at the center of the upper plate; however, when multiple chambers are provided and eccentric rotation is required, it may be positioned at one side of the upper plate rather than at its center.

In addition, the upper plate is preferably formed in a circular or elliptical shape, as shown in FIG. 3; however, it may also be manufactured in a rectangular or polygonal shape to accommodate the arrangement of internal magnets and chambers or for coupling with external systems. Furthermore, when an anti-separation means is provided on the central axis (120), the upper plate may be formed in a shape corresponding thereto to prevent separation between the upper and lower plates.

The chamber insertion portion may have an arcuate shape centered on the central axis (120). As described above, in the nanoparticle-based biochip for a rapid sterility test platform of the present invention, the position of the magnet (230) may be changed by the rotation of the upper plate. In this case, if the chamber insertion portion has the same shape as the chamber, the upper plate cannot rotate, and even if it is formed in a simple linear shape, rotation of the upper plate may be prevented. Therefore, when the chamber insertion portion has an arcuate shape as described above, the chamber can move along the arcuate shape, and as a result, the upper plate can rotate smoothly.

It is preferable that one chamber insertion portion be provided for each chamber. That is, in the present invention, since the sample chamber (131) and the control chamber (132) are provided on the lower plate, a sample chamber insertion opening (221) and a control chamber insertion opening (222) may be formed at positions corresponding to each chamber (see FIG. 2(a)).

In the present invention, as will be described below, since magnetic nanoparticles inside the chamber are moved by using a magnet (230), it is preferable that each chamber is provided with its own magnet (230) and chamber insertion port. If multiple chambers share a single chamber insertion port, the positions of the magnets (230) corresponding to each chamber may not be uniform, which can cause differences in the magnetic influence applied to each chamber and may lead to variations in observation results among the chambers.

A magnet (230) may be installed on one side of the chamber insertion port. The magnet (230) is used to collect the magnetic nanoparticles inside the chamber toward one side of the chamber, and it is preferable that the magnet (230) be installed on one side of the chamber insertion port.

That is, as the upper plate rotates, the distance between the chamber and the magnet changes. When the chamber is located at a position spaced apart from the magnet (230), the magnetic influence decreases, allowing the magnetic nanoparticles to remain suspended within the chamber. Conversely, when the chamber is located closer to the magnet (230), the magnetic nanoparticles may be collected toward one side of the chamber due to magnetic attraction (see Fig. 1).

In addition, in the present invention, the distance between the magnet and the chamber may change not only by the rotation of the upper plate but also by its linear movement. That is, when the chamber insertion port is formed in a linear shape, the upper plate moves linearly rather than rotationally, and in this case, the magnet mounted on the upper plate may also move linearly, thereby changing its distance from the chamber. In such a configuration, it is preferable that the upper plate be formed in a polygonal shape rather than a circular shape so as to facilitate the aforementioned linear movement.

This magnetic collection of the magnetic particles can provide two effects. The first effect is that it enhances the ease and accuracy of observation. As described above, the magnetic particles can bind to microorganisms to form complexes, and these complexes can be collected to one side of the chamber by the magnet (230). As a result, after the collection is completed, the concentration of microorganisms at the center of the chamber can become extremely low. Generally, when magnetic particles are used, a large amount of magnetic particles remain suspended inside the chamber, preventing light transmission and making it difficult to observe color or fluorescence changes caused by the indicator material. Therefore, in conventional methods, long-term incubation is required, after which the magnetic particles (complexes) are removed to observe turbidity or color changes. However, in the present invention, since the complexes are collected to one side of the chamber by the magnet as described above, even a slight change in color or fluorescence can yield a clear image.

The second effect is that it can promote the growth of microorganisms. In general, microorganisms stop proliferating once their concentration reaches a certain level. As discussed above, this is due to substrate competition, nutrient depletion, and external contamination; hence, maintaining the microorganism concentration below a certain threshold is necessary for sustained growth. In the present invention, this principle is utilized such that, when the upper plate is rotated forward to bring the chamber closer to the magnet (230), the concentration of complexes at the center of the chamber decreases, making image acquisition easier. Conversely, when the upper plate is rotated backward to increase the distance from the magnet (230), localized increases in microorganism concentration are alleviated, thereby accelerating the overall incubation rate. Accordingly, after image acquisition or when rapid incubation is required, the upper plate may be rotated backward to increase the gap between the magnet (230) and the chamber, whereas during image acquisition or when incubation is complete and further microbial culture is unnecessary, the upper plate may be rotated forward to reduce the distance between the chamber and the magnet.

Accordingly, even when the microorganism concentration is very low, by repeatedly performing image acquisition and incubation as described above, a high microorganism concentration can be achieved. Through this process, the sterility of a sample can be confirmed much more rapidly compared to conventional methods.

The magnet (230) may be installed on one side of the chamber insertion port. In particular, it is preferable that the magnet (230) be installed in the same direction as the rotational direction of the chamber (see Fig. 2). When the chamber is positioned apart from the magnet (230), it is desirable to minimize magnetic influence so that the magnetic nanoparticles remain suspended inside the chamber. When the chambers are arranged at equal intervals, if the rotational angle of each chamber is set to half the installation angle of the magnet (230), each chamber is positioned equidistantly from the respective magnets (230). In this case, the chamber is located between two magnets (230), and the magnetic nanoparticles inside the chamber experience minimal magnetic force and can remain suspended within the chamber (see Fig. 3).

When the magnet (230) is installed on either the outer or inner side of the rotational path of the chamber, the magnet (230) remains positioned on the outer or inner side during rotation, and as a result, the magnetic nanoparticles may be collected toward the inner or outer side of the chamber under the influence of the magnetic force.

A rotation means (240) may be installed on the outer surface of the upper plate to enable rotation of the upper plate. As described above, the distance between the chamber and the magnet (230) can be adjusted by the rotation of the upper plate. For this purpose, it is preferable that a rotation means (240) be installed on the outer surface of the upper plate. The rotation means (240) can rotate the upper plate forward or backward by transmitting power from a rotation driving unit (300) described later.

The rotation means (240) may be any structure capable of rotating the upper plate, but preferably it has a gear shape formed on the outer circumferential surface of the upper plate. That is, the upper plate of the present invention may have a cylindrical gear shape in which gear teeth are formed along the entire outer circumference.

Although it is preferable that the gear teeth be formed as spur gears, depending on the installation position of the rotation driving unit (300) described later, the gear may also be formed as a bevel gear or a screw gear, and it may also be formed as a helical gear to reduce noise.

The present invention also provides a rapid sterility testing platform including the biochip for a rapid sterility testing platform described above.

In one embodiment, the rapid sterility testing platform comprises: the biochip; a rotational drive unit for rotating the upper plate of the biochip; an illumination unit installed above or below the biochip; and an image acquisition unit for capturing the inside of the chamber of the biochip.

Since the biochip is the same as described above, detailed explanations thereof will be omitted.

The rotation driving unit (300) is a component that supplies power for rotating the upper plate and may be a gear connected to an electric motor. The gear may directly engage with the rotation means (240) of the upper plate to transmit power, or it may be connected through a chain, belt, or the like. It is preferable that the gear installed in the rotation driving unit (300) have a shape corresponding to the rotation means (240) of the upper plate. Specifically, the gear may be in the form of a spur gear, helical gear, rack gear, bevel gear, screw gear, or worm gear.

A lighting unit (400) may be installed above or below the biochip. The lighting unit (400) supplies illumination for observing the inside of the chamber. When observing color changes, white light may be used; when observing fluorescence, ultraviolet or visible light may be provided. In addition, the upper or bottom surface (110) of the chamber is preferably made of a transparent material to facilitate such illumination and observation.

Any light source capable of emitting light of a desired color may be used as the lighting unit; however, it is preferably an LED light source. The LED light source may emit visible or ultraviolet light, and a laser LED may also be used for fluorescence observation.

An image acquisition unit (500) may be installed to capture images of the interior of the chamber of the biochip. The image acquisition unit (500) is used to observe color or fluorescence changes inside the chamber and may employ an image acquisition device using a CCD. In the sterility test of the present invention, as described above, the color or fluorescence may change due to a redox indicator; therefore, by periodically capturing images with the image acquisition unit (500), the presence or absence of microorganisms can be easily determined. Furthermore, as previously described, to improve the accuracy of image acquisition, the upper plate may be rotated to bring the chamber closer to the magnet (230).

A more detailed description of the image acquisition unit (500) is as follows. For continuous time-lapse fluorescence imaging, the present invention may employ a miniaturized imaging system having dimensions of 224 mm × 120 mm × 140 mm. The imaging system includes an imaging area for the chip sample, an XYZ motorized stage for focus and positioning control, a 4× objective lens, a CCD camera, and may further include the lighting unit (400). Additionally, the imaging system of the present invention is preferably designed in a simplified form specialized for rapid sterility verification. This allows the system to be produced at a much lower cost and in a more compact configuration compared to commercially available equipment such as plate readers. Moreover, it can be easily installed in an incubator or the sterility test platform of the present invention, thereby facilitating temperature maintenance and space optimization.

Hereinafter, preferred embodiments of the present invention will be described with reference to the accompanying drawings so that those skilled in the art can easily practice them. Also, in describing the present invention, if it is determined that a detailed description of related known function or known configuration may obscure the gist of the present invention, the detailed description thereof will be omitted. In addition, certain features presented in the drawings have been enlarged or reduced or simplified for ease of explanation, and the drawings and their components are not necessarily drawn to proper scale. However, those skilled in the art will readily understand these details.

### Example 1

The separation and concentration of microorganisms using nanoparticles was conducted according to the procedure shown in Figure 3. Nanoparticles coated with a substance capable of binding to microorganisms were added in proportion to the sample volume of the biopharmaceutical to be tested, and the mixture was allowed to react for approximately 15 minutes at 35°C to induce binding between the nanoparticles and microorganisms. At this time, Apolipoprotein H, a type of protein, was used as the coating material for the nanoparticles. Subsequently, the sample was placed on a magnetic bar for 5 minutes to isolate the nanoparticles toward the wall, followed by removal of the supernatant except for the nanoparticles. Finally, a small amount of culture medium was added to the remaining sample containing the nanoparticles bound to microorganisms to resuspend the microorganisms. Specifically, Tryptic Soy Broth was used as the culture medium for aerobic microorganism detection, and Fluid Thioglycollate Medium was used for anaerobic microorganism detection.

The re-suspended sample was then injected into the sample chamber of the biochip, and resazurin, an oxidation- reduction indicator, was added at a concentration of 10 wt%. For the control chamber, the same concentrations of magnetic nanoparticles, culture medium, and resazurin were introduced as in the re-suspended sample.

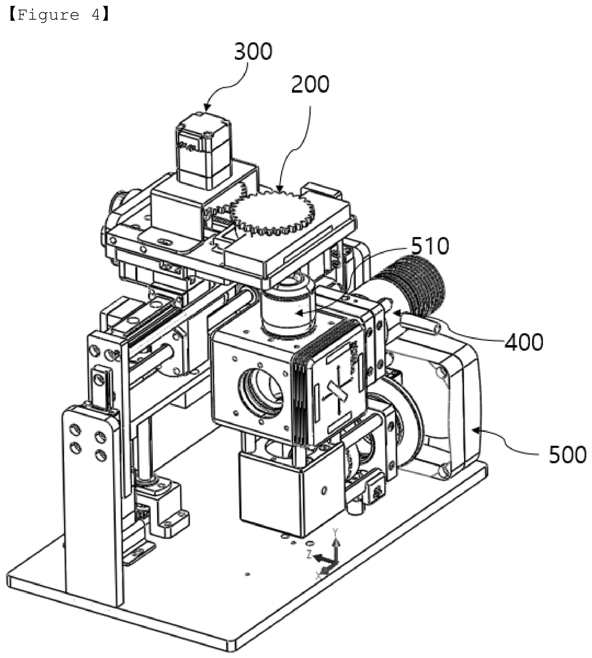

Next, the biochip was inserted into the imaging system shown in Figure 4, and fluorescence imaging was performed at predetermined intervals. The fluorescence intensity of the images obtained from both the sample and the control was measured using the analysis software, and the intensities were compared. If the fluorescence signal measured from the sample was significantly higher than that from the control, the sample was determined to be contamination-positive.

### Experimental Results

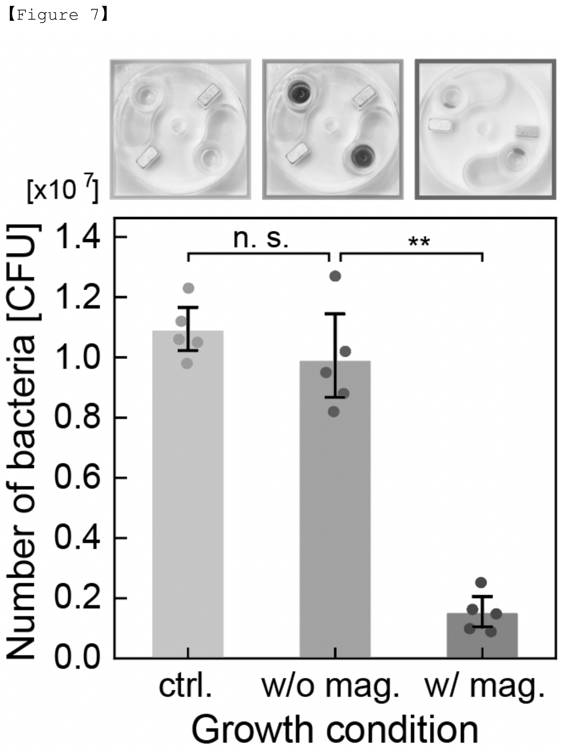

Figure 7 illustrates the experimental results confirming the performance of the biochip of the present invention. When the number of microorganisms after 12 hours of incubation was compared with the control (ctrl.), which was cultured in growth medium without nanoparticles, the condition where magnetic force was applied to aggregate the nanoparticles (w/ mag.) showed less than one-fifth the number of microorganisms. This indicates that microbial growth was suppressed due to the aggregated environment.
In contrast, when an environment simulating the removal of magnetic force (w/o mag.) was created using the biochip, the number of microorganisms observed after the same incubation time was similar to that of the control, which was statistically validated through five repeated experiments. Therefore, these results confirm that, in the present invention, the incubation rate can be regulated through a simple operation, such as rotation of the upper plate.

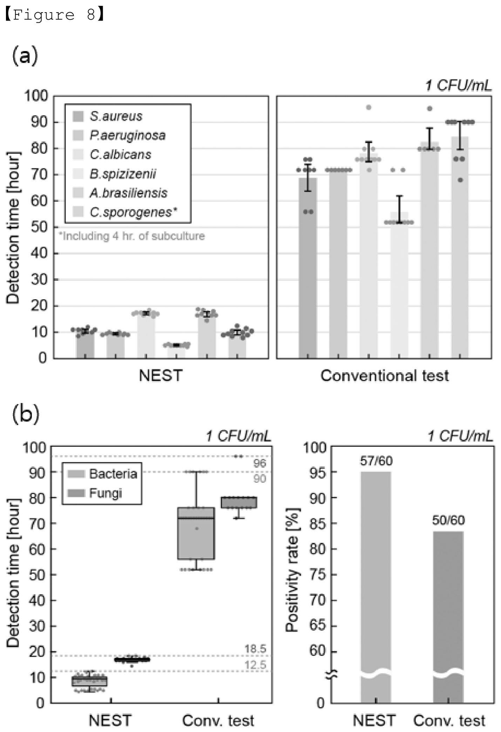

Figure 8 shows the comparison results between the rapid sterility verification test method (NEST) proposed by the present invention and the conventional 14-day incubation-based sterility test method. For six representative microorganisms recommended for sterility verification, 1 CFU/mL was introduced into the biopharmaceuticals, and both test methods were performed. Among 60 tests each, the rapid sterility verification test method showed an average of approximately 18-fold reduction in measurement time and superior positive detection accuracy compared to the conventional method. The rapid test detected 57 positive cases out of 60 (95% accuracy), whereas the conventional method detected 50 positive cases (83.3% accuracy).

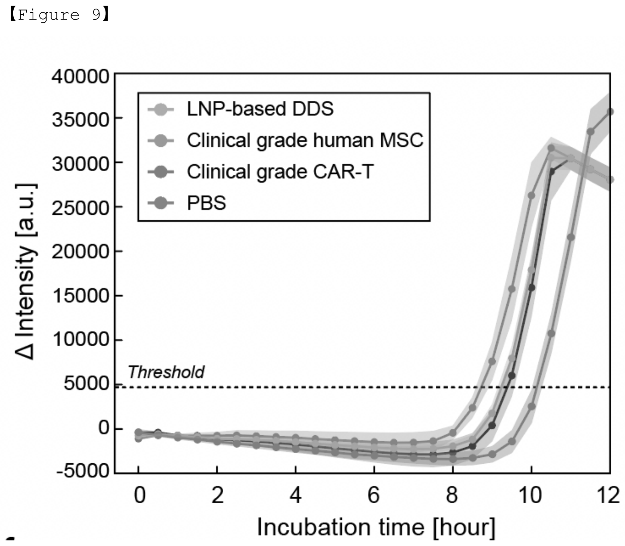

Figure 9 illustrates the rapid sterility verification test results for various types of biopharmaceuticals. Because the rapid sterility verification method selectively isolates only microorganisms, it can be universally applied regardless of the substances contained in the biopharmaceutical. To demonstrate this, a single microorganism type was introduced into various clinical-grade biopharmaceuticals, including CAR-T therapeutics, cell therapies, and RNA vaccines, and the rapid test was performed. All samples exhibited similar temporal signal variation patterns, confirming that only microbial signals were specifically detected and that the developed method possesses industrial scalability.

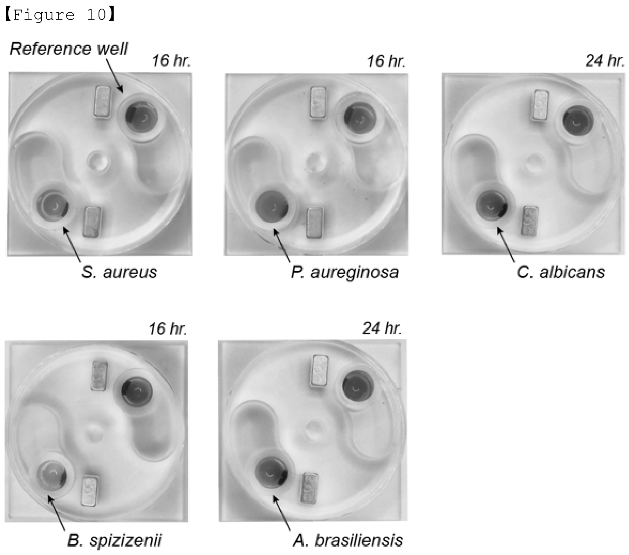

Additionally, the rapid sterility verification test of the present invention allows visual confirmation of microbial contamination without fluorescent imaging. As shown in Figure 10, although this method cannot be applied to anaerobic microorganisms and requires a longer time compared to fluorescence-based analysis, it enables convenient sterility testing even in settings without imaging equipment. For fast-dividing bacteria, contamination can be confirmed within 16 hours, and for slower-growing fungi, within 24 hours.

In the above, although specific parts of the content of the present invention have been described in detail, it will be clear to those skilled in the art that that these specific descriptions are merely preferred embodiments, and the scope of the present invention is not limited thereby. Accordingly, the substantial scope of the present invention will be defined by the appended claims and their equivalents.

## Claims

1. a rapid sterility testing method using a biochip including one or more chambers into which a sample and a control are respectively loaded, and one or more magnets installed at one side of each chamber, the method comprising:
mixing magnetic particles with a test subject material to bind a target analyte to the magnetic particles;
separating the magnetic particles from the test subject material to prepare a sample; and
determining the presence of the target analyte by injecting the separated sample and an indicator substance into the chamber of the biochip and microbial culture;
wherein the step of determining the presence of the target analyte comprises determining whether the target analyte is present in the sample by comparing fluorescence or color of images of the sample and the control after microbial culture.

2. The rapid sterility test method of claim 1, wherein the test subject material may be a biopharmaceutical, a cell therapy product, a clinical specimen, a chemical pharmaceutical product, a cosmetic, a food, or an environmental sample.

3. The rapid sterility test method of claim 1, wherein the target analyte is bacteria, mycoplasma, or fungi.

4. The rapid sterility test method of claim 1, wherein preparing the sample comprises concentrating the target analyte bound to the magnetic particles on a growth medium.

5. The rapid sterility test method of claim 4, wherein the growth medium comprises Tryptic Soy Broth, Muller-Hinton Broth, Fluid Thioglycollate Medium, Sabouraud Broth, or Luria-Bertani Broth.

6. The rapid sterility test method of claim 1, wherein determining the presence of the target analyte comprises:
spacing the chamber from the magnet;
injecting an indicator substance, sample, and control into the chamber and microbial culture for a predetermined time;
approaching the chamber to the magnet after completion of microbial culture; and
acquiring an image of the inside of the chamber and comparing acquired images of the separated sample and the control.

7. The rapid sterility test method of claim 6, wherein the target analyte is bound to the magnetic particles to form a complex, and the complex may be moved to one side of the chamber by the magnet when the chamber is brought into proximity with the magnet.

8. The rapid sterility test method of claim 1, wherein the microbial culture is performed for 4 to 48 hours.

9. The rapid sterility test method of claim 1, wherein image acquisition is performed at intervals of 10 to 120 minutes.

10. The rapid sterility test method of claim 1, wherein the indicator substance undergoes fluorescence or color change by the target analyte.

11. The rapid sterility test method of claim 10, wherein the indicator is a redox indicator substance, a pH indicator, a dye-based reagent, an enzyme reaction indicator, or an antibody-based reagent.

12. A biochip for a rapid sterility testing platform, comprising:
a lower plate including chambers into which the control and the sample are loaded; and
an upper plate engaged with the lower plate and including a magnet
wherein the lower plate comprises:
a bottom surface;
a central axis protruding upward from the bottom surface; and
one or more chambers installed at a predetermined distance from the central axis, and
the upper plate comprises:
a central axis insertion hole into which the central axis of the lower plate is inserted;
a chamber insertion hole into which the chamber is inserted; and
a magnet installed at one side of the chamber insertion hole.

13. The biochip of claim 12, wherein the chamber comprises one or more sample chambers and one or more control chambers.

14. The biochip of claim 13, wherein the chambers are installed at equal intervals at a uniform distance from the central axis.

15. The biochip of claim 12, wherein the chambers further contains magnetic particles and an indicator substance.

16. The biochip of claim 15, wherein the magnetic particles form a complex by binding with the target analyte.

17. The biochip of claim 16, wherein the magnetic particles include a functional group or substance capable of binding to microorganisms on their surface.

18. The biochip of claim 16, wherein the complex is positioned at one side of the chamber by the magnet during fluorescence or optical observation using the indicator substance.

19. The biochip of claim 12, wherein the upper plate is rotated at a predetermined angle around the central axis.

20. The biochip of claim 19, wherein the control chamber insertion hole has an arc shape around the central axis.

21. The biochip of claim 19, wherein the distance between the magnet and the chamber changes according to rotation of the upper plate.

22. The biochip of claim 19, wherein a rotation means for rotating the upper plate is installed on an outer surface of the upper plate.

23. A rapid sterility test platform comprising the biochip according to any one of claims 12 to 22.

24. The rapid sterility test platform of claim 23, comprising:
the biochip;
a rotational drive unit for rotating the upper plate of the biochip;
an illumination unit installed above or below the biochip; and
an image acquisition unit for capturing the inside of the chamber of the biochip.

25. The rapid sterility test platform of claim 24, wherein the image acquisition unit acquires fluorescence images or optical images of the inside of the chamber.

26. The rapid sterility test platform of claim 24, wherein the rapid sterility test platform determines the presence of microorganisms in the sample by comparing fluorescence or color between an initially acquired image and a subsequently acquired image.
